Europäisches Patentamt

European Patent Office

Office européen des brevets

(11) Publication number: **0 151 020**
**A2**

(12) # EUROPEAN PATENT APPLICATION

(21) Application number: **85300577.5**

(22) Date of filing: **29.01.85**

(51) Int. Cl.⁴: **A 61 B 10/00**

(30) Priority: **30.01.84 CA 446355**

(71) Applicant: **WALLACE BERESFORD SHUTE, 300 Island Park Drive, Ottawa Ontario, K1Y OA7 (CA)**

(43) Date of publication of application: **07.08.85 Bulletin 85/32**

(72) Inventor: **WALLACE BERESFORD SHUTE, 300 Island Park Drive, Ottawa Ontario, K1Y OA7 (CA)**

(74) Representative: **Bridge-Butler, Alan James et al, G.F. REDFERN & CO. High Holborn House 52/54 High Holborn, London WC1V 6RL (GB)**

(84) Designated Contracting States: **AT BE CH DE FR GB IT LI NL SE**

(54) **Surgical instrument.**

(57)    The invention relates to a two-piece disposable surgical instrument for collecting tissue cells for use as specimens for cytological diagnosis, in particular, the detection of cancer of the cervix and uterus.

Each part of the instrument is moulded from thermoplastic material. One part consists of a barrel having a reduced end having two finger rings at the other. The reduced end has a pair of scraping blades, portions of which serve as forward walls of cell collection areas.

The remaining part of the instrument consists of a plunger slidable within the barrel and having a thumb-grip, the plunger being capable of collecting and ejecting cells from the barrel and obtained from a different area of the cervix to those areas from which the pair of blades obtained their cells.

## SURGICAL INSTRUMENT

It is known from my Canadian Patent No. 614,878 which issued on February 21st 1961, to provide a surgical instrument (hereinafter termed "the patented instrument") for collecting tissue cells for use as specimens for cytological diagnosis, in particular, the detection of cancer of the cervix and uterus.

While the patented instrument has, in all respect, been admirable for the above purpose, one of the major drawbacks accompanying the use of metals - even stainless steel - in surgical instruments is the absolute necessity of sterilizing the instruments between uses. Furthermore, the instruments must also be sterilized immediately prior to use. Therefore, it is often necessary to sterilize the same instrument twice - once after use, to prevent the spread of bacteria in surgery, and again immediately prior to use to ensure that the patient is not exposed to any extraneous source of bacteria.

It is therefore necessary to maintain highly specialized, expensive sterilization equipment in a surgery, if metal instruments are to be used. It is also necessary to maintain a supply of these expensive instruments to ensure the smooth operation of a surgery.

Another drawback particularly associated with known metallic uterine cell collection instruments is the difficulty in ensuring that the rubber sealing rings of the plunger and the surrounding metallic areas are properly sterilized. These rings must also be periodically replaced because of wear.

I have found that by utilizing thermoplastic material in the manufacture of uterine cell collectors, it is practical to manufacture disposable cell collectors, which are sterilized and sealed in an ultra-clean environment. The examining physician is then able to remove the pre-sterilized instrument from its container, use it, and dispose of it, without being concerned that the used instrument will either spread bacteria in the surgery or pass any communicable disease to the next patient. The use of such plastic materials in manufacturing a uterine cell collector thereby serves not only to ensure complete and uniform cleanliness of the cell collector, but also compensates for the inefficiencies and time involved in in-surgery sterilization, and the onerous possiblility that a physician's sterilization equipment might malfunction.

The object of the present invention, therefore, is to provide such a surgical instrument that will overcome the above disadvantages and yet, will be relatively inexpensive to manufacture and of simple construction.

Accordingly, the present invention relates to a surgical instrument for collecting tissue cells for use as specimens for cytological diagnosis, said instrument being of the type having an elongated barrel provided with a pair of exterior scraping blades the longitudinal axes of which extend substantially normal to one another, and a plunger slidable within said barrel, said blades and said plunger being utilized for said collection, characterized in that (a) the instrument is disposable, being formed from thermoplastic material; (b) the longitudinal axis of the scraping edge of one of said blades extends at an angle of between $14^\circ$ and $16^\circ$ to the longitudinal axis of the scraping edge of the remaining

-4-

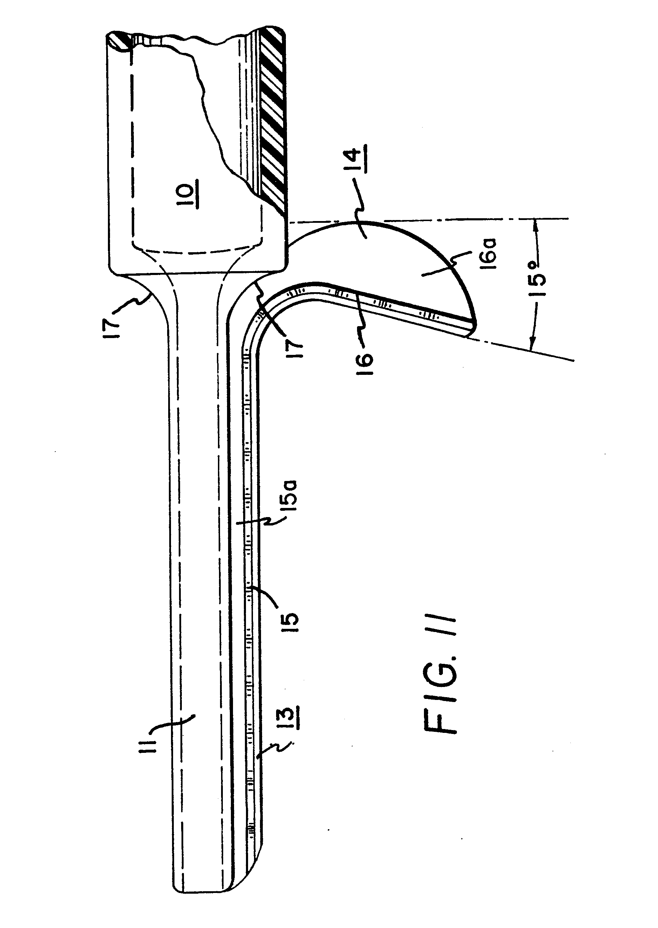

Figure 11 is an alternative embodiment of the barrel member.

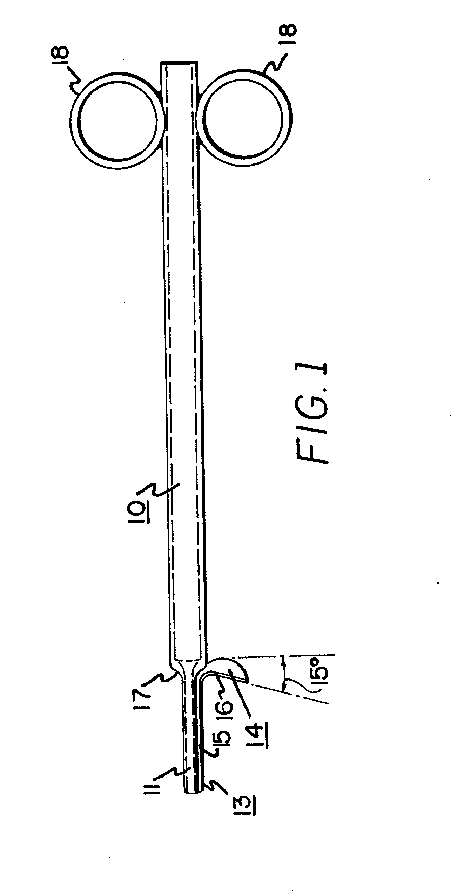

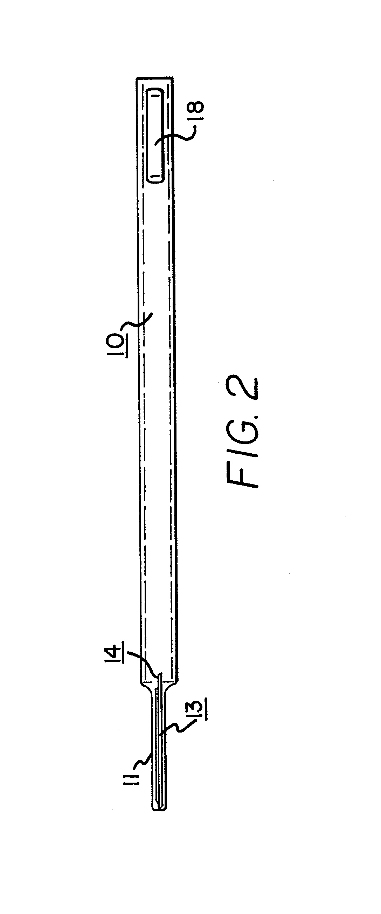

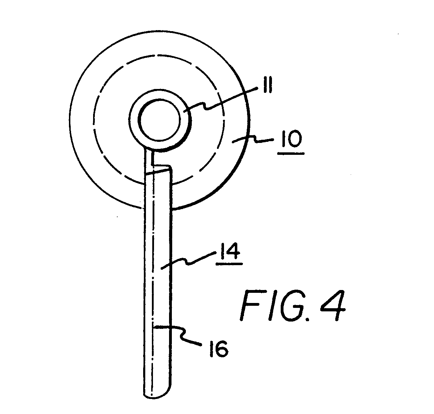

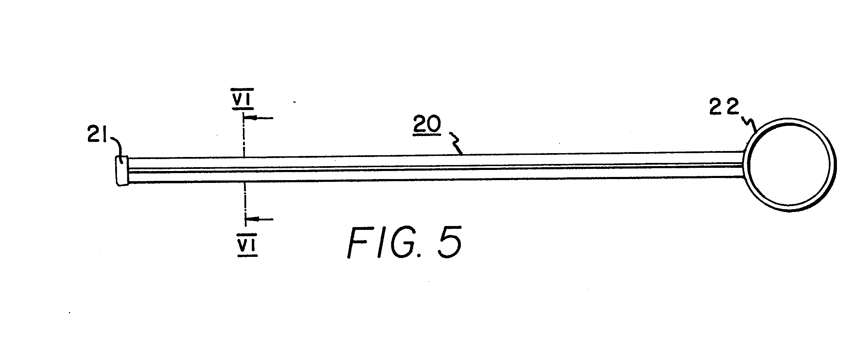

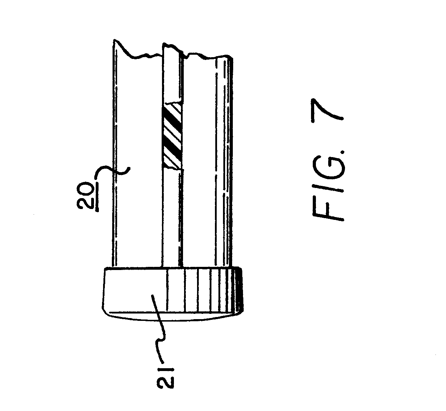

Referring to the drawings, the invention basically consists of two components i.e. an elongated barrel member shown in some detail in Figures 1 to 4 and a plunger member shown in some detail in Figures 5 to 7 and 9, both members being formed from thermoplastic material.

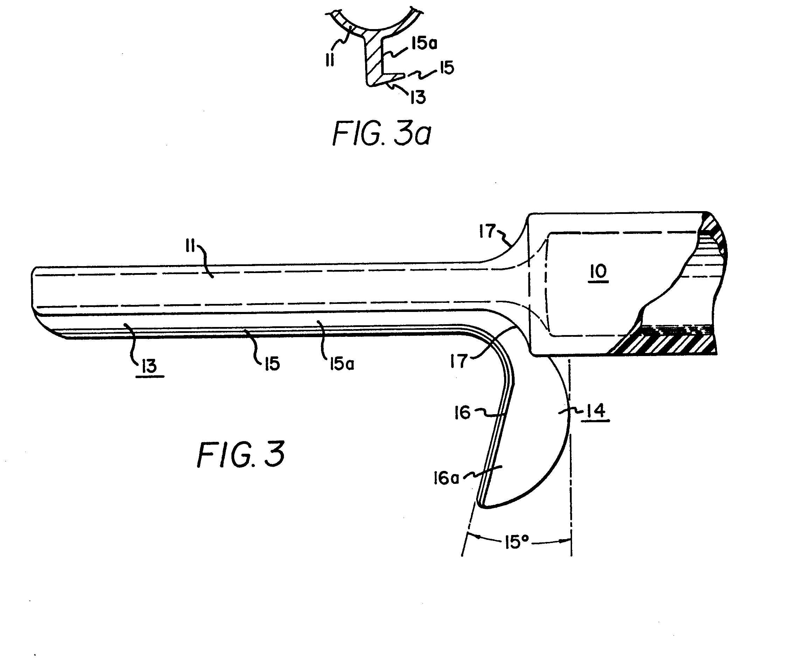

Referring specifically to Figures 1 to 4, the elongated barrel member or cylinder 10 has its intracervical portion 11 considerably reduced in diameter and is provided with a first or endocervical scraping blade (indicated generally at 13) extending throughout its length having a longitudinal axis, said first blade gradually merging into a second or portio vaginalis scraping blade, indicated generally at 14, projecting from adjacent the distal end of the barrel 10. The operative edge 15 (shown more clearly in Figure 3a) of the first blade 13 extends parallel with the longitudinal axis of said distal end 11 whereas the operative edge 16 of the second blade 14 extends at an angle of between $14^{\circ}$ and $16^{\circ}$ (Figures 1 - 3) to the longitudinal axis of blade 14 and is, in fact, preferably at an angle of $15^{\circ}$.

As will be seen more clearly from reference to Figures 3 and 3a the operatives edges 15, 16 of the scraping blades 13, 14 respectively, each serve as a forward boundary wall of an associated cell collection area, 15a, 16a respectively, the purpose of which will be described hereinafter. The longitudinal axes of the blades 13 and 14 extend substantially normal to one another.

blade so as to obtain maximal approximation of the cutting edge of said one blade to the medial portion of the portio vaginalis that communicates with the endocervical canal; and (c) the distal end of the barrel is inwardly curved towards its intracervical portion so as to accommodate that portion of said portio vaginalis in frictional contact therewith.

The invention is illustrated, by way of example, in the accompanying drawings in which:

Figure 1 is a longitudinal view of the barrel member;

Figure 2 is a plan view of Figure 1;

Figure 3 is a detailed view, on an enlarged scale, of the distal end of the barrel member;

Figure 3a is a detailed cross-section, on an enlarged scale, of one of the scraping blades;

Figure 4 is an end view of Figure 3;

Figure 5 is a longitudinal view of the plunger member;

Figure 6 is a transverse section, on an enlarged scale, taken on the line VI – VI of Figure 5;

Figure 7 is a detailed view, on an enlarged scale, of the distal end of the plunger member;

Figure 8 is an alternative embodiment of the plunger member;

Figure 9 is a longitudinal section of the assembled instrument;

Figure 10 is a diagrammatic view of the surgical instrument and its relation to the cervix during collection of tissue cells; and

-5-

It will also be seen that the distal end of the barrel 10 is inwardly curved, as at 17, towards said intracervical portion 11. Moreover, said proximal end of the barrel member 10 is provided with a pair of diametrically opposed finger-rings 18.

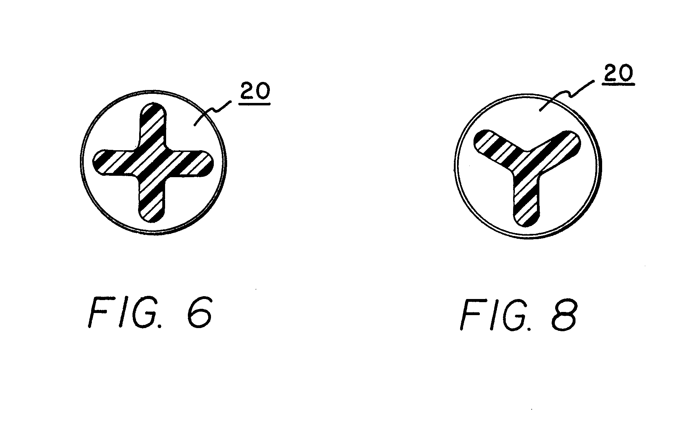

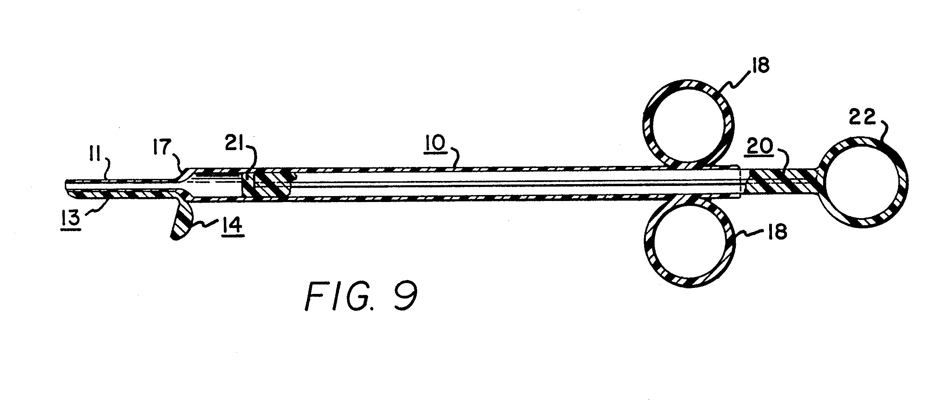

Referring now to Figures 5 - 7 which illustrate the preferred form of the plunger member, indicated generally at 20, its major length has a cross-section which is substantially cruciform in cross-section which can be more easily seen from Figure 6. The distal end of the plunger member 20 is provided with a slightly domed plunger head 21 which is adapted to be in slidable frictional contact with the inner periphery of the barrel member 10 while its proximal end is provided with a thumb-grip 22. If desired, the major length of the plunger member 20 could be of substantially triangular cross-section as is shown in Figure 9.

The completely assembled surgical instrument is shown in partial longitudinal section in Figure 9.

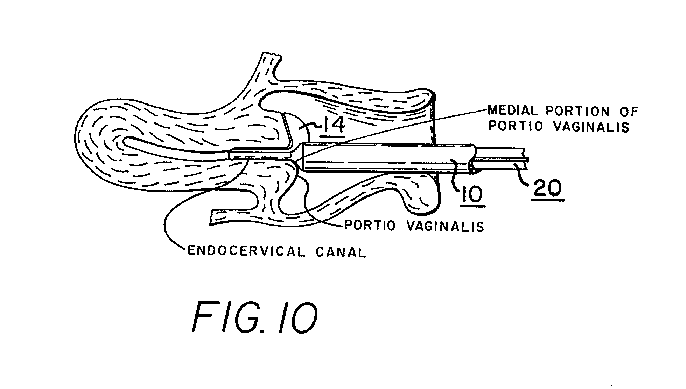

The operation of the instrument is such that the examining physician first exposes the cervix with a vaginal speculum. The distal end of the instrument is then inserted into the vagina so that the end portion 11, as well as the first blade 13, is located within the cervical canal of the uterus, the said end portion 11 being adjacent to the internal os of the cervix. At the same time, the second blade 14 is thus located adjacent to, and gently engaging, the portio vaginalis of the cervix. The physician then, as was the case in the patented instrument, rotates the instrument forming the subject of instant application clockwise through 360° with continuous pressure upon the entire wall of the cervical canal and the vaginal portio so that the operative edge 15 of the first blade 13 obtains a comprehensive sampling of cells from the endocervix which are deposited on cell collection area 15a. Simultaneously, the operative edge 16 of the second blade 14 obtains a representative sampling of cells from the squamocolumnar junction and the adjacent surface of the portio vaginalis and which cells are deposited on cell collection area 16a. Immediately prior to the complete withdrawal of the instrument, the operator suddenly applies traction to the plunger member 20 by means of the thumb-grip 22, whereby the domed plunger head 21 sucks cellular debris and mucus from the uterine cavity above the level of the internal os of the cervix into the interior of the intracervical portion 11 of the barrel member. Figure 10 shows diagrammatically the position of the instrument and its relation to the uterus and cervix when samples are required to be taken from the three locations just mentioned.

After the instrument has been withdrawn from the patient, the plunger 20 is depressed forcefully so that its domed head 21 ejects the said cellular debris from the barrel to enable a smear to be made and, of course, the said scrapings are removed from the collection areas 15a and 16a so as to enable similar smears to be made when they are dropped into a fixative solution and prepared for cytologic examination.

Although, blades 13 and 14 are shown in Figures 1 - 4 and 9 and 10 as non-serrated, if desired, their operative edges may be serrated as is shown in greater detail in Figure 11.

From experience, it has been found that the preferred

angle of 15° of the second blade 14 approximates far more to the natural surface angle or contour of the portio vaginalis than did that of the similiar blade of the patented instrument thus giving a much improved surface engagement.

The instrument is preferably moulded from any suitable thermoplastic material such as, for example, both high and low density polyethylene, or polypropylene, or acrylonitrile-butadiene-styrene (A.B.S. resin), rubber-modified polystyrene, cellulose acetate, or cellulose acetate butyrate.

-8-

THE EMBODIMENTS OF THE INVENTION IN WHICH AN EXCLUSIVE PROPERTY OR PRIVILEGE IS CLAIMED ARE DEFINED AS FOLLOWS:-

1.  A surgical instrument for collecting tissue cells for use as specimens for cytological diagnosis, said instrument being of the type having an elongated barrel provided with a pair of exterior scraping blades the longitudinal axes of which extend substantially normal to one another, and a plunger slidable within said barrel, said blades and said plunger being utilized for said collection, characterized in that:

(a)  the instrument is disposable, being formed from thermoplastic material;

(b)  the longitudinal axis of the scraping edge of one of said blades extends at an angle of between $14^{\circ}$ and $16^{\circ}$ to the longitudinal axis of the scraping edge of the remaining blade so as to obtain maximal approximation of the scraping edge of said one blade to the medial portion of the portio vaginalis that communicates with the endocervical canal; and

(c)  the distal end of the barrel is inwardly curved towards its intracervical portion so as to accommodate that portion of said portio vaginalis in frictional contact therewith.

2.  An instrument according to Claim 1 wherein the operative edge of each said blades serves as a forward boundary wall of an associated cell collection area.

3.  An instrument according to Claim 2 wherein the operative edge of each said blade is planar.

-9-

4.      An instrument according to Claim 2 wherein the operative edge of each said blade is serrated.

5.      An instrument according to Claim 2 wherein said thermoplastic material is selected from the group consisting of high and low density polyethylene, or polypropylene, or acrylonitrile-butadiene-styrene (A.B.S. resin), rubber-modified polystyrene, cellulose acetate, or cellulose acetate butyrate.

6.      An instrument according to Claim 5 formed of a moulded thermoplastic material.

FIG.1

FIG. 2

FIG. 3a

FIG. 3

15°

FIG. 4

VI

22

20

21

FIG. 5

VI

FIG. 7

FIG. 6

FIG. 8

0151020

FIG. 9

MEDIAL PORTION OF
PORTIO VAGINALIS

~14

10    20

PORTIO VAGINALIS

ENDOCERVICAL CANAL

FIG. 10

FIG. 11